Europäisches Patentamt

European Patent Office

Office européen des brevets

(19)

(11) Veröffentlichungsnummer: **0 035 593**

**A2**

(12)

# EUROPÄISCHE PATENTANMELDUNG

(21) Anmeldenummer: 80107003.8

(22) Anmeldetag: 13.11.80

(51) Int. Cl.³: **A 61 K 9/20**

(30) Priorität: 01.03.80 DE 3007878

(43) Veröffentlichungstag der Anmeldung:
16.09.81 Patentblatt 81/37

(84) Benannte Vertragsstaaten:
AT CH FR GB LI

(71) Anmelder: SIGRI ELEKTROGRAPHIT GMBH
Werner von Siemens-Strasse 18
D-8901 Meitingen(DE)

(72) Erfinder: Stübner, Gerhard, Dr.
AK St. Georg Lohmühlenstrasse 5
D-2000 Hamburg 1(DE)

(72) Erfinder: Wolter, Dietmar, PD Dr.
Ihlendieksweg 30
D-2070 Grosshansdorf(DE)

(54) Verwendung von Kohlenstoff als implantierbarer Träger und Träger aus Kohlenstoff.

(57) Aus Kohlenstoff bestehendes Implantat in Form einer Prothese oder eines Hilfskörpers, das Träger eines durch Imprägnieren eingebrachten Antibiotikums ist. Die Aktivität des Antibiotikums wird durch den biokompatibelen Träger nicht beeinträchtigt und die Desorption des Antibiotikums wird durch Strukturierung der Trägeroberfläche den jeweiligen Anforderungen angepaßt.

EP 0 035 593 A2

Croydon Printing Company Ltd.

- 1 -

Träger für Antibiotika

Die Erfindung betrifft einen Träger für Antibiotika.

Die Verwendung von Antibiotika enthaltenden geformten Körpern aus Knochenzement, z.B. bei der Therapie von Knocheneiterungen oder Abszeßhöhlen, ist bekannt. Knochenzemente bestehen im wesentlichen aus einem oder mehreren härtbaren Harzen, vor allem Methylmethacrylat-, Methacrylat-, Methacrylsäuremethyl- Homo- und Copolymeren, die durch Zusatz eines Monomeren plastifiziert werden. Das Gemisch härtet dann unter Wärmeentwicklung aus, wobei der Temperaturanstieg so groß sein kann, daß zugesetzte Antibiotika inaktiviert werden.

Es ist ebenfalls bekannt, aus Kohlenstoff und Graphit bestehende Implantate zu verwenden, z.B. als Ersatz für Gelenke oder Bänder oder auch als Reizelektrode. Die Implantate werden zu einem Teil mit einem Knochenzement verankert, dessen antibiotische Wirksamkeit wegen der oben beschriebenen Veränderungen der dem Zement zugesetzten Antibiotika häufig nicht befriedigt. Zu einem anderen Teil werden Implantate ohne die Zugabe von Antibiotika direkt mit dem Knochengewebe verschraubt oder in Bindegewebe eingelegt. In beiden Fällen lassen sich Infektionen nicht immer ausschließen.

- 2 -

Der Erfindung liegt daher die Aufgabe zugrunde, eine Verwendungsform von Antibiotika zu schaffen, bei der die Aktivität eines Antibiotikums nicht beeinträchtigt wird und der Träger des Antibiotikums biokompatibel ist.

Die Aufgabe wird gelöst mit einem Träger aus Kohlenstoff, wobei unter dem Begriff "Kohlenstoff" auch Graphit zu verstehen ist.

Die Träger aus Kohlenstoff sind dabei als Prothese oder als reine, nur für eine begrenzte Frist im Körper verbleibende Hilfskörper ausgebildet. Im letzten Fall besteht der Träger zweckmäßig aus einer, einen größeren Porenanteil aufweisenden Kohlenstoffsorte, die zur Beladung mit einem Antibiotika in eine antibiotikahaltige Lösung getaucht wird. Die Aufnahme der Lösung kann in bekannter Weise durch Evakuieren des Trägers vor dem Eintauchen und gegebenenfalls durch Erhöhen des Drucks nach dem Eintauchen des Trägers in die Lösung beschleunigt werden. Gegebenenfalls kann der imprägnierte Träger auch durch schnelle Temperatursenkung einer Behandlung ähnlich dem Gefriertrocknen unterworfen werden. Gleichzeitig als Prothese verwendete Träger weisen im allgemeinen eine sehr kleine Porosität auf, so daß die durch Imprägnierung aufgenommene Menge an Antibiotika im allgemeinen nicht ausreicht. Bevorzugt werden in diesem Fall Träger, deren Oberfläche eine mikroporöse Struktur aufweist. Die mikroporöse Struktur wird durch partielle Oxidation des Kohlenstoffs erzeugt, z.B. durch Erhitzen des Trägers in Luft oder einer wasserstoff- oder kohlendioxidhaltigen Atmosphäre. Die Oberfläche des aktivierten Trägers wird dann durch Tauchen in eine antibiotikahaltige Lösung mit einer im wesentlichen aus Antibiotika bestehenden Schicht belegt. Bei Gelenkprothesen aus Kohlenstoff kann es dabei von Vorteil sein, mechanisch

- 3 -

weniger belastete Teile der Prothese mit einer größeren Porosität zu versehen, als mechanisch hochbelastete Teile. Hüftgelenkendoprothesen können zum Beispiel im Bereich des Kragens eine verhältnismäßig hohe Porosität und entsprechend nach dem Imprägnieren eine hohe Konzentration an Antibiotika aufweisen. Die hohe, sich am Gelenkshämatom aufbauende Antibiotikakonzentration ist dabei von besonderem Vorteil.

Träger aus Kohlenstoff können schließlich auch im direkten Kontakt mit Knochenzement verwendet werden, da wegen der günstigen Wärmeleitfähigkeit des Kohlenstoffs die beim Härten des Zements freiwerdende Wärme abgeleitet und gleichmäßig in der Masse verteilt wird, so daß die Trägertemperatur nicht auf ein Antibiotika schädigendes Niveau ansteigt. Zweckmäßig verwendet man in diesem Fall als Träger Kohlenstoffasern, in der Form von Kurzschnittfasern oder textilen Flächengebilden, wie Gewebe oder Gewirken, die gleichzeitig Festigkeit und Schrumpfung des Zements verbessern. Die Oberflächen der Kohlenstoffasern werden wie oben dargestellt mit einer mikroporösen Struktur versehen und mit einer Antibiotika enthaltenden Schicht belegt. Geeignete Verfahren zum Einmischen von Kohlenstoffasern in einen Knochenzement sind beispielsweise in DE-OS 29 47 839 und 29 47 885 beschrieben.

Bei einer anderen Anwendungsform sind Kohlenstoffträger in Form kleiner Kügelchen mit einer Schicht aus Knochenzement versehen und gegebenenfalls zu mehreren auf Metall- oder Kunstharzfäden aufgereiht. Über die Dicke der Zementschicht läßt sich die Desorption der Antibiotika von der Kohlenstoffoberfläche steuern und zugleich die Festigkeit des Gesamtsystems erhöhen, bis etwa eine für das Herausziehen des Systems nötige Mindestfestigkeit erreicht ist.

- 4 -

Die Im- und Explantierbarkeit wird auch durch Einführen kugelförmiger Trägerteilchen in biegsame Polyamidfaser-Schläuche erleichtert.

Es bedarf keiner weiteren Erläuterung, daß die mit einem Antibiotikum beladenen Träger sich auch mit Vorteil in Verbindung mit anderen Implantatwerkstoffen verwenden lassen, z.B. mit Kunststoffen oder auch Metallen.

Erfindungsgemäße Träger aus Kohlenstoff sind vollständig inert, so daß an den Träger gebundene Antibiotika nicht durch chemische und physikalische Veränderungen geschädigt werden. Ein anderer wesentlicher Vorteil besteht darin, daß die Antibiotika verhältnismäßig langsam von der Trägeroberfläche desorbiert werden, und somit über längere Zeit wirksam bleiben, wie das folgende Beispiel erläutert:

Graphitwürfel mit einer Kantenlänge von 8 mm, die eine Porosität von etwa 20 % und einen maximalen Porendurchmesser von etwa 0,05 mm aufweisen, wurden in eine physiologische Kochsalzlösung getaucht, die 4 mg Gernebcin/ml Lösung enthielt. Die Tauchzeit betrug 24 h. Die Träger wurden getrocknet, 72 h in physiologischer Kochsalzlösung eluiert und dann wieder getrocknet. Der Zyklus wurde mehrmals wiederholt und jeweils nach der Eluierung die Antibiotikakonzentration mit dem Agar-Diffusions-Loch-Test bestimmt. Die antibiotische Wirksamkeit blieb dabei über mehrere Wochen unverändert.

– 1 –

Patentansprüche:

1. Verwendung von Kohlenstoff als implantierbaren Träger für Antibiotika.

2. Träger aus Kohlenstoff nach Patentanspruch 1,
dadurch gekennzeichnet,
daß der Träger eine Oberfläche mit mikroporöser Struktur aufweist.